(19) Europäisches Patentamt
European Patent Office
Office européen des brevets

(11) EP 1 616 553 B1

(12) **FASCICULE DE BREVET EUROPEEN**

(45) Date de publication et mention de la délivrance du brevet:
**18.04.2007 Bulletin 2007/16**

(21) Numéro de dépôt: **05291113.8**

(22) Date de dépôt: **24.05.2005**

(51) Int Cl.:
***A61K 8/06*** *(2006.01)* ***A61K 8/92*** *(2006.01)*
***A61K 8/37*** *(2006.01)* ***A61K 8/39*** *(2006.01)*
***A61K 8/02*** *(2006.01)* ***A61Q 17/04*** *(2006.01)*
***A61Q 1/00*** *(2006.01)* ***A61Q 1/14*** *(2006.01)*
***A61Q 1/10*** *(2006.01)* ***A61Q 1/04*** *(2006.01)*

(54) **Emulsion huile-dans-eau fine contenant un filtre hydrophile**

Feine Emulsion vom Typ Öl-in-Wasser die einen hydrophilen UV-Absorber enthält

Oil-in-water fine emulsion containing a hydophilic filter

(84) Etats contractants désignés:
**AT BE BG CH CY CZ DE DK EE ES FI FR GB GR HU IE IS IT LI LT LU MC NL PL PT RO SE SI SK TR**

(30) Priorité: **28.06.2004 FR 0451344**

(43) Date de publication de la demande:
**18.01.2006 Bulletin 2006/03**

(73) Titulaire: **L'ORÉAL**
**75008 Paris (FR)**

(72) Inventeurs:
- **Guiramand, Carole**
  **78350 Jouy en Josas (FR)**
- **Caplain, Dominique**
  **75014 Paris (FR)**

(74) Mandataire: **Rasson, Catherine**
**L'OREAL - D.I.P.I.**
**25-29 Quai Aulagnier**
**92600 Asnières (FR)**

(56) Documents cités:
**EP-A- 0 667 144**
**EP-A- 1 120 102**
**WO-A-02/064107**
**US-A- 5 498 406**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 616 553 B1

## Description

La présente invention se rapporte à une composition pour application topique sous forme d'émulsion huile-dans-eau fine, contenant au moins un filtre hydrophile, obtenue sans apport d'énergie, son procédé de préparation et son utilisation notamment pour le traitement, le soin, le maquillage et/ou le nettoyage de la peau, des phanères (cheveux, cils, ongles) et/ou des muqueuses. La composition peut être notamment une composition cosmétique et/ou dermatologique.

Pour diverses raisons liées en particulier à un meilleur confort d'utilisation (douceur, émollience et autres), les compositions cosmétiques ou dermatologiques actuelles se présentent le plus souvent sous la forme d'une émulsion du type huile-dans-eau (H/E), c'est à dire un support constitué d'une phase continue dispersante aqueuse et d'une phase discontinue dispersée huileuse. Ces émulsions H/E sont plus demandées que les émulsions du type eau-dans-huile (E/H), qui sont constituées d'une phase continue dispersante grasse et d'une phase discontinue dispersée aqueuse, car elles apportent sur la peau à l'application, un toucher plus doux, moins gras et plus léger que les systèmes d'émulsions E/H.

Par ailleurs, en cosmétique, en dermatologie et en pharmacie, les émulsions comportant des globules (ou gouttelettes) de la phase dispersée de petite taille, appelées aussi émulsions fines, sont souvent recherchées que ce soit :

- pour leur texture : les compositions peuvent être plus ou moins visqueuses et aller de la consistance d'une lotion à celle d'une crème ;
- pour leur aspect visuel qui peut varier d'une composition transparente ou opalescente à une composition blanche ;
- pour leur toucher cosmétique favorisant notamment leur rapidité de pénétration ;
- pour leur large positionnement possible en terme de marché, de telles compositions satisfaisant les consommateurs aussi bien de l'Europe que du Japon et d'autres pays.

On entend dans la présente demande par « émulsion fine » une émulsion dont la taille des globules de la phase dispersée va de 50 à 500 nanomètres.

En terme de technologie, à l'heure actuelle pour obtenir ce type d'émulsions H/E fines, il faut apporter de l'énergie au mélange, soit une énergie mécanique importante afin de fragmenter la phase dispersée en fines globules, soit une énergie thermique en passant par un procédé de changement de phase avec la température (80°C), comme les systèmes préparés par technique PIT « Phase Inversion Température ». Ces systèmes sont bien connus et permettent aujourd'hui de disposer d'émulsions fines.

Ainsi, la technique d'apport d'énergie mécanique permet d'obtenir des émulsions fines transparentes, encore appelées « nanoémulsions », décrites par exemple dans les documents EP-A-728460, EP-A-780114, EP-A-780115, EP-A-879589, EP-A-1,010,413, EP-A-1,010,414, EP-A-1,010,415, EP-A-1,010,416, EP-A-1,013,338, EP-A-1,016,453, EP-A-1,018,363, EP-A-1,020,219, EP-A-1,025,898, EP-A-1,120,102, EP-A-1,120,101, EP-A-1,160,005, EP-A-1,172,077 et EP-A-1,353,629. Les globules d'huile des nanoémulsions ont une taille moyenne inférieure à 100 nm. L'inconvénient de ces nanoémulsions est la nécessité de faire intervenir une énergie mécanique importante.

Des nanoémulsions sont décrites aussi dans les publications de Forgiarini, J. Esquena, C. Gonzàlez and C. Solans, « Formation of Nano-emulsions by Low Energy Emulsification. Methods at Constant Temperature », Langmuir, 2001, 17, 2076-2083H., et de Forgiarini, J. Esquena, C. Gonzàlez and C. Solans, « Studies of the Relation Between Phase Behavior and Emulsification Methods with Nanoemulsion Formation », Prog. Colloid Polym Sci., 2000, 115 (Trends in Colloid and Interface Science XIV), 36-39. Ces publications décrivent des émulsions décane-dans-eau stabilisées par un tensioactif particulier, le laureth-4 (ou Brij 30), et réalisées par addition d'eau dans un mélange décane/Brij 30. Le tensioactif comporte une chaîne alkyle courte (C12), ce qui le rend plus irritant que ses homologues à chaîne alkyle plus longue. Par ailleurs, les émulsions décrites dans ces documents sont instables, notamment sur le plan microscopique (diamètre de gouttes), et de ce fait trop instables pour une application industrielle.

Par ailleurs, la technique par PIT est, dans son principe, bien connue de l'homme de l'art et elle est notamment décrite dans les articles "Phase Inversion Emusification", de Th Förster et al, paru dans Cosmetics & Toiletries, vol. 106, Décembre 1991, pp 49-52, «Application of the phase-inversion-température method to the emulsification of cosmetics» de T. MITSUI et al, paru dans American Cosmetics and Perfumery, vol 87, Décembre 1972, et dans les documents WO-A-89111907, DE-A-4318171, EP-A-815846 et EP-A-1,297,824.

Toutefois, ces techniques d'obtention d'émulsions fines présentent des inconvénients suivants :

- la température élevée du procédé PIT induit des contraintes en terme de formulation. Ainsi, il est difficile d'utiliser cette technique avec des molécules sensibles à la chaleur et ayant des points éclairs bas, et la technique est donc limitée aux molécules insensibles à la chaleur et présentant des points éclair élevés. Cela restreint le type et le nombre de matières premières utilisables, ou bien, si on veut utiliser par exemple des molécules ayant des points éclairs bas, il faut, en fonction de ces matières premières, adapter le mode opératoire, et l'obtention de ces émulsions

devient alors plus complexe et plus coûteuse. De ce fait, ce procédé exclut ou du moins limite l'utilisation de composés volatils tels que les composés lipophiles volatils, notamment les huiles volatiles comme les silicones volatiles, et de certains actifs ou extraits végétaux thermosensibles.

- les homogénéisateurs en haute ou très haute pression, qui permettent de réaliser des émulsions fines par apport d'énergie sont des matériels coûteux et fragiles, donc générant des coûts importants de mises en oeuvre industrielles.

Par ailleurs, on connaît dans l'état de la technique des microémulsions transparentes. Les microémulsions ne sont pas à proprement parler des émulsions, contrairement aux nanoémulsions ; ce sont des solutions transparentes de micelles gonflées par de l'huile, cette huile étant en général à chaîne très courte (ex : hexane, décane) et étant par ailleurs solubilisée grâce à la présence conjointe d'une quantité importante de tensioactifs et de co-tensioactifs formant les micelles. La taille des micelles gonflées est très petite en raison de la faible quantité d'huile qu'elles peuvent solubiliser. Cette très petite taille des micelles est la cause de leur transparence. Cependant, contrairement aux nanoémulsions décrites ci-dessus, les microémulsions se forment spontanément par mélange des constituants sans apport d'énergie mécanique autre qu'une simple agitation magnétique et quel que soit l'ordre d'ajout des constituants. En outre, ce sont des systèmes thermodynamiquement stables. Les inconvénients majeurs des microémulsions sont liés à leur forte proportion en tensioactifs par rapport à l'huile, conduisant à des intolérances et entraînant un toucher collant lors de l'application sur la peau. Par ailleurs, l'état de microémulsion du système est défini par le choix des constituants et leurs proportions relatives, ainsi que la température, comme le montre le diagramme de phases présenté à la figure 11.7 de l'article « The colloidal domain, D.F. Evans, H. Wennerström, Editeur Wiley-VCH (1999). Pour la description des microémulsions, on peut se reporter par exemple à l'article de M. Bourrel et R.S. Schechter « Microemulsions and related systems », pages 25 à 30, Ed. Marcel Denker, 1988. Ces microémulsions ne sont donc pas des émulsions fines et ne peuvent pallier les inconvénients des émulsions fines décrites ci-dessus.

En outre, quand on souhaite réaliser une composition de protection solaire, il est nécessaire d'incorporer des filtres dans les émulsions, et c'est dans ce cas d'autant plus difficile de faire des émulsions fines que les filtres sont des facteurs de déstabilisation des dites émulsions.

Il subsiste donc le besoin de réaliser des émulsions H/E fines contenant des filtres hydrophiles, qui restent stables si on les dilue et qui soient obtenues avec des procédés moins coûteux et moins complexes que ceux de l'art antérieur, c'est à dire des procédés ne nécessitant pas d'apport d'énergie, que cette énergie soit mécanique ou thermique, et donc sans passer par des températures élevées ou sans matériel apportant beaucoup d'énergie, ces procédés n'ayant pas d'incidence sur la stabilité chimique des composés constituant la composition.

La demanderesse a trouvé de façon surprenante la possibilité de réaliser des émulsions fines contenant des filtres, sans apport d'énergie et réalisées entièrement à température ambiante, grâce à un choix particulier de tensioactifs, à un choix particulier d'huiles, à un rapport huiles/tensioactif spécifique, et à un choix particulier de filtres.

La présente invention a donc pour objet une composition pour application topique sous forme d'émulsion huile-dans-eau, comprenant une phase huileuse dispersée dans une phase aqueuse, dont les globules de phase huileuse ont un diamètre moyen allant de 50 à 500 nm, caractérisée en ce que :

- elle contient un système émulsionnant contenant (i) au moins un tensioactif non ionique, ayant un point de fusion inférieur à 45°C et un HLB allant de 10 à 15, ledit tensioactif comprenant une partie polaire comportant au moins 5 groupes oxyéthylénés et une partie apolaire comportant au moins une chaîne alkyle ramifiée ou insaturée, ayant de 14 à 22 atomes de carbone, et (ii) au moins un tensioactif anionique,
- elle contient au moins un filtre UV hydrophile,
- la phase huileuse contient des constituants huileux dont au moins une huile hydrocarbonée de poids moléculaire égal ou supérieur à 400, la quantité d'huiles hydrocarbonées de poids moléculaire égal ou supérieur à 400 représentant au moins 25 % en poids par rapport au poids total de la phase huileuse et la quantité d'huiles à base de triglycérides représentant moins de 15 % en poids par rapport au poids total de la phase huileuse,
- le rapport en poids de la quantité des constituants huileux de la phase huileuse sur la quantité du système émulsionnant va de 0,8 à 3,5.

On entend ici par « application topique » une application externe sur les matières kératiniques, que sont notamment la peau, le cuir chevelu, les cils, les sourcils, les ongles, les cheveux et/ou les muqueuses. La composition étant destinée à une application topique, elle comprend un milieu physiologiquement acceptable. On entend par « milieu physiologiquement acceptable », un milieu compatible avec la peau, les lèvres, le cuir chevelu, les cils, les yeux, les ongles et/ou les cheveux. La composition peut constituer notamment une composition cosmétique ou dermatologique.

On entend dans la présente demande par « quantité des constituants huileux de la phase huileuse » la quantité totale des huiles et autres corps gras présents dans la phase huileuse, c'est-à-dire la quantité des constituants de la phase huileuse autres que le système émulsionnant comprenant notamment les tensioactifs et co-tensioactifs.

On entend par ailleurs par « filtre hydrophile », un composé minéral ou organique, capable de filtrer les radiations solaires, ce composé étant hydrosoluble ou hydrodispersible, c'est-à-dire pouvant se solubiliser dans l'eau à une concentration de 0,01 % en poids. Un composé est considéré comme non hydrophile si sa solubilité dans l'eau est inférieure à 0,01 % en poids.

Selon une caractéristique essentielle des compositions conformes à la présente invention, le diamètre moyen des globules (ou gouttelettes) de phase huileuse dispersée dans la phase aqueuse dispersante va de 50 à 500 nm, de préférence de 50 nm à 250 nm et plus particulièrement de 80 à 200 nm, ce diamètre moyen étant un diamètre moyen en intensité, mesuré par diffusion quasi-élastique de la lumière, par exemple par l'appareil Model BI-90 de la société Brookhaven Instruments Corporation.

Selon la taille des gouttelettes de phase huileuse, l'aspect visuel de la composition selon l'invention va de la transparence à un aspect blanc en passant par l'opalescence.

Les émulsions selon l'invention présentent l'avantage de pouvoir être préparées à température ambiante et donc par un procédé qui ne dégrade pas les constituants de la composition (actifs...), qui, en outre, est peu onéreux, peu complexe et sans contrainte puisque aucune énergie n'est nécessaire pour l'obtention de ce système. Les émulsions selon l'invention présentent de plus l'avantage d'être parfaitement stables dans le temps et en température puisque aucun crémage (c'est-à-dire remontée des globules d'huile), aucune sédimentation (c'est-à-dire rassemblement des globules d'huile au fond du récipient), et aucun déphasage (c'est-à-dire séparation des phases aqueuse et huileuse) n'est observé dans le temps et à différentes températures de stockage (4°C, 25°C et 45°C). Les émulsions selon l'invention présentent encore l'avantage de pouvoir se présenter de manière très variée, c'est-à-dire en ayant une large gamme de texture en terme de viscosité, de toucher (en fonction du taux d'huile par exemple), ce qui permet de les proposer à des consommateurs ayant des habitudes et des sensibilités très différentes.

En outre, les compositions contenant les filtres hydrophiles selon l'invention présentent l'avantage d'être photoprotectrices tout en étant faciles à étaler et agréables à appliquer (sensation de fraîcheur), et d'être parfaitement stables dans le temps et en température comme indiqué ci-dessus.

Les compositions selon l'invention peuvent être plus ou moins visqueuses et avoir l'aspect allant de la lotion (produit fluide) à la crème (produit épais). Ainsi, leur viscosité peut aller par exemple de 1 cpoise (1 mPa.s) à 20.000 cpoises (20.000.mPa.s), viscosité mesurée après 10 minutes de cisaillement, à l'aide du viscosimètre Rhéomat 180 à une vitesse de cisaillement de 200 rpm, au mobile adapté à la viscosité de la composition.

Système émulsionnant

La composition selon l'invention contient un système émulsionnant contenant (i) au moins un tensioactif non ionique et (ii) au moins un tensioactif anionique. Dans ce système émulsionnant, le tensioactif non ionique est généralement introduit dans la phase huileuse alors que le tensioactif anionique peut être introduit soit dans la phase aqueuse soit dans la phase huileuse.

Le système émulsionnant peut être présent en une quantité allant de 0,6 à 11 % en poids, de préférence de 1,1 à 9 % en poids par rapport au poids total de la composition.

1. Tensioactif non ionique

Les tensioactifs non ioniques utilisés dans la composition de l'invention ont un point de fusion inférieur à 45°C et sont donc liquides à une température inférieure à 45°C, et notamment ils sont liquides à une température allant de 20 à 44°C. Ils ont un HLB allant de 10 à 15, et ils comportent une partie polaire comportant au moins 5 groupes oxyéthylénés et une partie apolaire comportant au moins une chaîne alkyle ramifiée ou insaturée, ayant de 14 à 22 atomes de carbone.

On calcule la balance HLB (balance hydrophile-lipophile) d'un tensioactif émulsionnant suivant la formule suivante :

$$HLB = 20 \times \frac{\text{m hydrophile}}{\text{m totale du TA}}$$

dans laquelle m hydrophile représente le poids du groupement hydrophile (soit la partie polaire) et m totale du TA représente le poids total du tensioactif.

Selon un mode préféré de réalisation de l'invention, les tensioactifs non ioniques sont choisis parmi les esters d'acide gras et de polyéthylène glycol, les esters de polyol et d'acide gras oxyéthylénés, les éthers d'alcool gras oxyéthylénés, et leurs mélanges. Ces tensioactifs oxyéthylénés comportent au moins 5 groupes oxyéthylénés ; ils peuvent comporter par exemple de 5 à 21 groupes oxyéthylénés et de préférence de 5 à 18 groupes oxyéthylénés.

Comme esters d'acide gras et de polyéthylène glycol, on peut citer par exemple le PEG-8 isostearate (ou isostéarate de polyéthylène 400) tel que le produit commercialisé sous le nom Prisorine 3644 par la société UNIQEMA, le PEG-10 isostearate, le PEG-12 isostearate, le PEG-15 isostearate, et leurs mélanges.

Comme esters de polyol et d'acide gras oxyéthylénés, on peut citer notamment les esters de glycéryle et d'acide gras oxyéthylénés, les esters de sorbitol et d'acide gras oxyéthylénés, comme par exemple le PEG-15 glyceryl isostearate tel que le produit commercialisé sous le nom Oxypon 2145 par la société Zschimmer Schwarz, le PEG-20 sorbitan triisostearate, et leurs mélanges.

Comme éther d'alcool gras oxyéthylénés, on peut citer par exemple l'isosteareth-10 tel que le produit commercialisé sous le nom Arosurf 66E10 par la société Witco.

On peut utiliser un mélange des tensioactifs cités ci-dessus.

La quantité de tensioactif(s) non ionique(s) tels que définis ci-dessus peut aller par exemple de 0,5 à 10 % en poids et de préférence de 1 à 8 % en poids par rapport au poids total de la composition.

2. Tensioactif anionique

Les tensioactifs anioniques utilisés dans la composition de l'invention peuvent être plus particulièrement choisis dans le groupe formé par :

- les sels alcalins du dicetyl- et du dimyristylphosphate ;
- les sels alcalins du cholestérol sulfate ;
- les sels alcalins du cholestérol phosphate ;
- les sels d'acylaminoacides (ou lipoaminoacides) tels que les acylglutamates mono- et di-sodiques comme le sel disodique de l'acide N-stéaroyl L-glutamique commercialisé sous la dénomination Acylglutamate HS21 par la société AJINOMOTO ;
- les sels de sodium de l'acide phosphatidique ;
- les dérivés alkyl sulfates et alkyl sulfonates ;
- et leurs mélanges.

Selon un mode préféré de réalisation de l'invention, on utilise comme tensioactif anionique, notamment un sel d'acylaminoacide tel que les acylglutamates mono- et di-sodiques comme le sel disodique de l'acide N-stéaroyl L-glutamique commercialisé sous la dénomination Acylglutamate HS21 par la société AJINOMOTO.

Les tensioactifs anioniques peuvent être présents dans l'émulsion de l'invention, en une quantité allant de préférence de 0,05 à 2 % en poids et plus particulièrement de 0,1 à 1 % en poids par rapport au poids total de la composition.

3. Co-tensioactifs

Le système émulsionnant peut comprendre aussi un ou plusieurs co-tensioactifs, qui sont des tensioactifs de HLB inférieure à 5. Ces co-tensioactifs ont une température de fusion inférieure ou égale à 45°C et sont donc liquides à une température inférieure ou égale à 45°C ; ils sont notamment liquides à une température de 20 à 45°C. Comme co-tensioactifs, on peut citer par exemple les alcools gras tels que l'alcool isostéarylique, l'alcool oléique ; les esters d'alcool gras et de glycérol tels que l'isostéarate de glycérol ; les esters d'alcool gras et de sorbitan, comme l'isostearate de sorbitan. La quantité de co-tensioactifs peut aller par exemple de 0,005 à 5 % en poids et de préférence de 0,01 à 2 % en poids par rapport au poids total de la composition.

Filtres UV

Les filtres UV hydrophiles sont choisis parmi les filtres UV organiques, les agents filtrants minéraux (pigments ou nanopigments d'oxydes métalliques -titane, fer, zinc, zirconium ou cérium - enrobés ou non, solubles ou dispersibles dans l'eau), et leurs mélanges.

Les filtres UV organiques peuvent être ioniques ou non-ioniques, et/ou être des polymères.

Selon un mode préféré de réalisation de l'invention, les filtres UV hydrophiles utilisés selon l'invention sont choisis parmi les dérivés du phényl benzimidazole, les dérivés de la benzophénone, les dérivés du phényl benzotriazole et leurs mélanges.

a) Comme dérivés du phényl benzimidazole, on peut citer par exemple l'acide phényl benzimidazole sulfonique,

vendu notamment sous le nom commercial Eusolex 232 par la société Merck, qui a la structure chimique suivante :

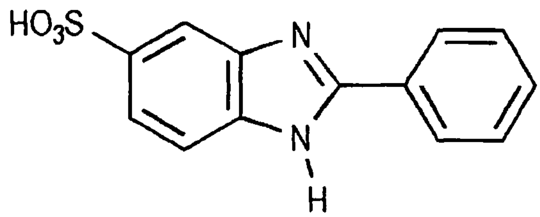

On peut aussi citer le benzimidazilate vendu notamment sous le nom commercial Neo Heliopan AP par la société Haarmann et Reimer.

b) Comme dérivés de la benzophénone, on peut citer par exemple la benzophénone 4, vendue notamment sous le nom commercial Uvinul MS40 par la société BASF, qui a la structure chimique suivante :

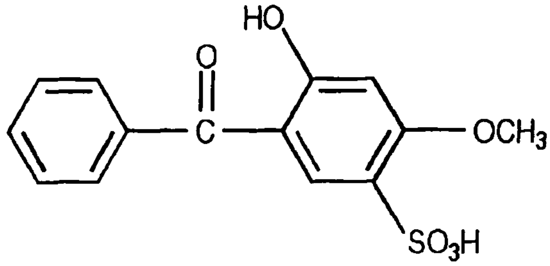

On peut aussi citer la benzophenone 9 commercialisée sous le nom Uvinul DS49 par la société BASF.

c) Comme dérivés du phényl benzotriazole, on peut citer par exemple le méthylène bis-benzotriazolyl tetramethylbutylphénol, vendu notamment sous le nom commercial de Tinosorb M par la société Ciba Specialty Chemicals, qui a la structure chimique suivante :

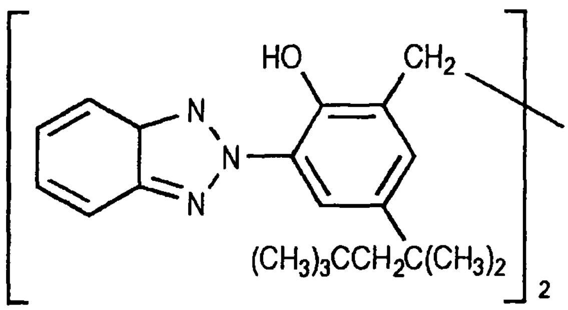

On peut utiliser un mélange de ces filtres.

Comme filtres hydrophiles inorganiques, on peut citer par exemple la dispersion aqueuse d'oxyde de titane anatase (60 nm) enrobé de silice/alumine, commercialisée sous le nom Mirasun TIW60 par la société Rhodia, l'oxyde de titane (rutile) micronisé traité silice / hydroxyde d'aluminium / acide alginique commercialisée sous le nom MT-100AQ par la société Tayca.

La quantité de filtre(s) hydrophile(s) varie suivant la protection solaire recherchée et notamment le SPF (ou indice de protection solaire) souhaité. Cette quantité (en matière active) peut aller par exemple de 0,1 à 20% en poids par rapport au poids total de la composition.

La composition selon l'invention est préférentiellement exempte de filtres lipophiles (c'est-à-dire hydrophobes), ceux-ci ayant tendance à déstabiliser la composition.

Phase huileuse

La phase huileuse comporte des constituants huileux, et elle doit contenir au moins une huile hydrocarbonée de poids moléculaire égal ou supérieur à 400, la quantité d'huiles hydrocarbonées de poids moléculaire égal ou supérieur à 400 représentant au moins 25 % en poids par rapport au poids total de la phase huileuse, et par exemple de 25 à 100 % en poids de la phase huileuse, mieux de 25 à 80 % en poids et encore mieux de 30 à 70 % en poids de la phase huileuse.
Par ailleurs, la phase huileuse doit contenir moins de 15 % en poids d'huiles à base de triglycérides par rapport au poids total de la phase huileuse.
On entend par « huile hydrocarbonée » dans la présente demande, une huile formée essentiellement, voire constituée, d'atomes de carbone et d'hydrogène, et éventuellement d'atomes d'oxygène, d'azote, et ne contenant pas d'atome de silicium ou de fluor. Une telle huile peut contenir des groupes ester, éther, amine, amide. Les huiles hydrocarbonées de poids moléculaire égal ou supérieur à 400, utilisées selon l'invention sont choisies parmi les alcanes ayant un point de fusion inférieur à 45°C, les esters d'acide gras, les éthers d'alcool gras, et leurs mélanges. On peut citer en particulier comme huiles hydrocarbonées de poids moléculaire égal ou supérieur à 400, l'huile de jojoba ; les esters d'acide gras tels que le palmitate d'isocétyle, le stéarate d'isocétyle ; les huiles d'origine végétale ; les éthers d'alcools gras tels que le diisostéaryl éther. Selon un mode préféré de réalisation de l'invention, au moins une huile hydrocarbonée de poids moléculaire égal ou supérieur à 400 est choisie parmi les alcanes ayant un point de fusion inférieur à 45°C.
Comme alcanes ayant un point de fusion inférieur à 45°C, on peut citer notamment le polyisobutène hydrogéné tel que l'huile de Parléam®, l'huile de vaseline, et leurs mélanges. Selon un mode particulier de réalisation de l'invention, la quantité d'alcanes de point de fusion inférieur à 45°C peut représenter de 25 à 100 % en poids de la phase huileuse, mieux de 25 à 80 % en poids et encore mieux de 30 à 70 % en poids de la phase huileuse.
Les huiles à base de triglycérides ont généralement un poids moléculaire supérieur à 400. Toutefois, pour atteindre le but de l'invention, la quantité de ces huiles doit être limitée à moins de 15 % en poids et de préférence à moins de 10 % en poids par rapport au poids total de la phase huileuse, la phase huileuse comprenant, comme indiqué ci-dessus, les constituants huileux sans les tensioactifs du système émulsionnant. Comme huiles à base de triglycérides, on peut citer les huiles d'origine végétale telles que l'huile d'amande douce, l'huile d'avocat, l'huile de ricin, l'huile d'olive, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle.
En plus des huiles hydrocarbonées de poids moléculaire égal ou supérieur à 400, la phase huileuse peut contenir en outre une ou plusieurs huiles de poids moléculaire inférieur à 400, choisies par exemple parmi les huiles de silicone ou les huiles hydrocarbonées de poids moléculaire inférieur ou égal à 400. Comme huiles de silicone, on peut citer notamment les huiles de silicone cycliques ou linéaires, notamment celles ayant une viscosité inférieure ou égale à 10 centistokes à 25°C comme les cyclopolydiméthylsiloxanes (cyclométhicones) telles que la cyclopentasiloxane et la cyclohexadiméthylsiloxane. Comme huiles hydrocarbonées ayant un poids moléculaire inférieur à 400, on peut citer par exemple les isoparaffines telles que l'isododécane (poids moléculaire : 194), l'isohexadécane (poids moléculaire : 258), le dioctylcyclohexane ; les esters d'acides gras comme le myristate d'isopropyle, le palmitate d'isopropyle, l'éthylhexyl palmitate, l'isostéaryl néopentanoate, l'isononyl isononanoate (poids moléculaire : 320) ; les éthers gras tels que le dicaprylyl éther.
La quantité de phase huileuse peut aller par exemple de 0,5 à 55 % en poids par rapport au poids total de la composition, de préférence de 1 à 40 % en poids, mieux de 2 à 40 % en poids et encore mieux de 5 à 30 % en poids par rapport au poids total de la composition.
Dans la composition selon l'invention, le rapport en poids de la quantité des constituants huileux de la phase huileuse sur la quantité du système émulsionnant va de 0,8 à 3,5 et de préférence de 0,7 à 3. Comme indiqué ci-dessus, la quantité des constituants huileux de la phase huileuse correspond à la quantité totale des huiles et autres corps gras présents dans la phase huileuse, c'est-à-dire la quantité des constituants de la phase huileuse autres que les tensioactifs et/ou co-tensioactifs faisant partie du système émulsionnant.

Phase aqueuse

De manière classique, la phase aqueuse dispersante peut être constituée par de l'eau, ou un mélange d'eau et de composés hydrophiles, tels que notamment les alcools polyhydriques comme par exemple la glycérine, le propylène glycol, le dipropylène glycol et le sorbitol, les alcools inférieurs hydrosolubles tels que l'éthanol, l'isopropanol ou le butanol. En outre, elle peut bien entendu contenir des adjuvants hydrosolubles ou hydrodispersibles, et en particulier les adjuvants hydrosolubles cosmétiques ou dermatologiques classiquement utilisés.
La phase aqueuse peut représenter de 45 à 99,5 % en poids par rapport au poids total de la composition, de préférence de 60 à 99 % en poids par rapport au poids total de la composition et mieux de 60 à 98 % en poids par

rapport au poids total de la composition.

Adjuvants

Parmi les adjuvants susceptibles d'être contenus dans la phase aqueuse et/ou dans la phase huileuse des émulsions conformes à l'invention (selon leur caractère hydrosoluble ou liposoluble), on peut citer notamment les épaississants ioniques ou non ioniques, les antioxydants, les émollients, les actifs cosmétiques ou dermatologiques, les parfums, les conservateurs, les charges, les séquestrants, les pigments, les colorants, ou tout autre ingrédient habituellement utilisé dans les domaines considérés.

Bien entendu, l'homme de l'art veillera à choisir le ou les éventuels composés à ajouter à la composition selon l'invention et leurs quantités de manière telle que les propriétés avantageuses attachées intrinsèquement à la composition conforme à l'invention ne soient pas, ou substantiellement pas, altérées par l'addition envisagée.

Comme actifs, on peut citer par exemple :

- les agents hydratants tels que par exemple le lactate de sodium ; les polyols, et en particulier la glycérine, le sorbitol, les polyéthylène glycols ; le mannitol ; les acides aminés ; l'acide hyaluronique ; la lanoline ; l'urée et les mélanges contenant de l'urée, tels que le NMF (« Natural Moisturising Factor ») ; la vaseline ; l'acide N-lauroyl pyrrolidone carboxylique et ses sels ; les acides gras essentiels ; les huiles essentielles ; et leurs mélanges.
- les actifs anti-vieillissement et les agents kératolytiques tels que les a-hydroxy-acides et notamment les acides dérivés de fruits, comme les acides glycolique, lactique, malique, citrique, tartrique, mandélique, leurs dérivés et leurs mélanges ; les β-hydroxy-acides comme l'acide salicylique et ses dérivés tels que l'acide n-octanoyl-5-salicylique ou l'acide n-dodécanoyl-5-salicylique ; les a-céto-acides comme l'acide ascorbique ou vitamine C et ses dérivés tels que ses sels comme l'ascorbate de sodium, l'ascorbylphosphate de magnésium ou de sodium ; ses esters comme l'acétate d'ascorbyle, le palmitate d'ascorbyle et le propionate d'ascorbyle, ou ses sucres comme l'acide ascorbique glycosylé, et leurs mélanges ; les β-céto-acides ; les rétinoïdes comme le rétinol (vitamine A) et ses esters, le rétinal, l'acide rétinoïque et ses dérivés, ainsi que les rétinoïdes décrits dans les documents FR-A-2,570,377, EP-A-199636, EP-A-325540, EP-A-402072 ; l'adapalène ; les caroténoïdes ; et leurs mélanges.
- les vitamines, comme les vitamines A et C indiquées ci-dessus, et aussi comme la vitamine E (tocophérol) et ses dérivés ; la vitamine B3 (ou vitamine PP ou niacinamide) et ses dérivés ; la vitamine B5 (ou panthénol ou alcool panthénylique ou 2,4-dihydroxy-N-(3-hydroxypropyl)-3,3diméthylbutanamide), sous ses différentes formes : D-panthénol, DL-panthénol), et ses dérivés et analogues, tels que le panthoténate de calcium, la panthétine, la pantothéine, l'éther de éthyl panthényl, l'acide pangamique, la pyridoxine, le pantoyl lactose, et les composés naturels en contenant tels que la gelée royale ; la vitamine D et ses analogues tels que ceux décrits dans le document WO-A-00/26167 ; la vitamine F ou ses analogues tels que les mélanges d'acides insaturés possédant au moins une double liaison et notamment les mélanges d'acide linoléique, d'acide linolénique et d'acide arachidonique, ou les composés en contenant ;
- les antibactériens et anti-séborrhéiques tels que l'acide salicylique, le 2,4,4'-trichloro-2'-hydroxy diphényl éther (ou triclosan), le 3,4,4'-trichlorobanilide (ou triclocarban), l'acide azélaïque, le peroxyde de benzoyle et les sels de zinc comme le lactate, le gluconate, le pidolate, le carboxylate, le salicylate et/ou le cystéate de zinc.

Comme épaississants, on peut citer notamment les polymères épaississants, en particulier :

- Les polymères carboxyvinyliques, tels que les produits commercialisés sous les dénominations Carbopol (nom INCI : carbomer) par la société Noveon ; les polyacrylates et polyméthacrylates tels que les produits vendus sous les dénominations de Lubrajel et Norgel par la société Guardian ou sous la dénomination Hispagel par la société Hispano Chimica ;
- Les polyacrylamides ;
- Les polymères et copolymères d'acide 2-acrylamido-2-méthylpropane-sulfonique, éventuellement réticulés et/ou neutralisés, comme le poly(acide 2-acrylamido 2-méthylpropane sulfonique) commercialisé par la société CLARIANT sous la dénomination Hostacerin AMPS (nom INCI : ammonium polyacryldimethyltauramide) ou comme les copolymères réticulés d'acrylamide et d'AMPS, se présentant sous la forme d'une émulsion E/H, tels que ceux commercialisés sous le nom de SEPIGEL 305 (nom INCI : Polyacrylamide / C13-14 Isoparaffin / Laureth-7) et sous le nom de SIMULGEL 600 (nom INCI : Acrylamide / Sodium acryloyldimethyltaurate copolymer / Isohexadecane / Polysorbate 80) par la société SEPPIC. On peut citer aussi les polymères d'AMPS comportant une partie hydrophobe, réticulés ou linéaires, tels que les produits commercialisés sous les dénominations Aristoflex SNC, LNC et HMS par la société Clariant ;
- Les polysaccharides comme la gomme de xanthane, la gomme de guar et ses dérivés tels que l'hydroxypropyl guar, en particulier celle commercialisée sous le nom Jaguar HP105 par la société Rhodia, la gomme de caroube, la

gomme d'acacia, les scléroglucanes, les dérivés de chitine et de chitosane, les carraghénanes, les gellanes, les alginates, les celluloses telles que la cellulose muicrocristalline, la carboxyméthylcellulose, l'hydroxyméthylcellullose, l'hydroxypropylcellulose et l'hydroxyéthylcellulose, comme le produit commercialisé sous le nom Natrosol 250HHR par la société Aqualon ;

- Les polymères non ioniques comportant au moins une séquence hydrophobe et au moins une séquence hydrophile, tels que les polyuréthannes commercialisés sous les dénominations SERAD FX1010, SERAD FX1100 (nom INCI : Steareth-100/PEG-136/HMDI Copolymer) et le SERAD FX1035 par la société HÜLS, ceux commercialisés sous les dénominations Rheolate 255, Rheolate 278 et Rheolate 244 par la société RHEOX (nom INCI : Polyether-urea-polyurethane), ceux commercialisés sous les dénominations DW 1206F, DW 1206J, DW 1206B, DW 1206G par la société Röhm & Haas (nom INCI : Polyurethane), et celui commercialisé sous la dénomination Acrysol RM 2020 de la société Röhm & Haas. On peut citer aussi les solutions aqueuses de copolymère de SMDI et alcool gras, commercialisées sous les dénominations Aculyn 46 (nom INCI : PEG-150 stearyl alcohol/SMDI Polymer), et Aculyn 44 (nom INCI : PEG-150 decyl alcohol/SMDI Polymer) par la société Röhm & Haas ;
- Les polymères anioniques comportant au moins une chaîne hydrophobe, et notamment les polymères ou copolymères (y compris terpolymères) acryliques ou méthacryliques comportant au moins une chaîne hydrophobe, tels que les copolymères obtenus par copolymérisation d'acide acrylique ou méthacrylique ou leurs esters avec avec un monomère à insaturation éthylénique comportant un groupement hydrophobe, comme les copolymères réticulés commercialisés sous les noms PEMULEN TR1, PEMULEN TR2 ou CARBOPOL 1382 (nom INCI : Acrylates/C10-30 alkyl acrylate crosspolymer) par la société Noveon ; le terpolymère acide methacrylique/methyl acrylate/ dimethyl meta-isopropenyl benzyl isocyanate d'alcool éthoxylé, en solution aqueuse à 25 % commercialisé sous la dénomination Viscophobe DB1000 par la société Amerchol, le copolymère acide acrylique/itaconate de mono-stearyle oxyéthyléné (20 OE) en dispersion aqueuse à 30% commercialisé sous la dénomination Structure 2001 par la société National Starch, le copolymère acide acrylique/ itaconate de monocétyle éthoxylé (20 OE) à 30% en dispersion aqueuse commercialisé sous la dénomination Structure 3001 par la société National Starch, le copolymère acrylique soluble en milieu alcalin en dispersion aqueuse a 30 % commercialisé sous la dénomination Aculyn 22 par la société Röhm & Haas ;
- Les polymères cationiques contenant au moins une séquence hydrophobe et au moins une séquence hydrophile, tels que le Polyquaternium 24 comme le produit commercialisé sous la dénomination Quatrisoft LM200 par la société Amerchol ;
- Les polymères cationiques réticulés comme le polyquaternium-37 commercialisé sous les noms Salcare SC96 par la société Ciba et Synthalen CR par la société 3V Sigma.

L'invention a également pour objet un procédé de préparation de ces émulsions. Le procédé consiste :

(1) à préparer la phase huileuse (A) contenant la ou les huiles et autres corps gras, et le système émulsionnant, sous agitation, l'agitation étant faite par exemple avec un barreau aimanté, à une température allant environ de 20°C à 45°C, jusqu'à obtention d'une phase homogène,

(2) à introduire dans la phase (A) 0.1 à 3 % en poids d'eau (phase B) par rapport au poids total de la composition, et à mélanger jusqu'à obtention d'une phase homogène (C),

(3) à ajouter dans la phase (C), 55% à 75% en poids d'eau (phase D) par rapport au poids total de la composition, pour obtenir après mélange une phase E homogène, et

(4) à ajouter le reste des constituants de la phase aqueuse (phase F).

Le ou les filtres UV hydrophiles se trouvent généralement dans la phase F.

L'agitation se fait de préférence avec un barreau aimanté ou tout autre système d'agitation donnant une faible agitation et donc de faible énergie, à une température pouvant aller de 20° à 45°C. On entend par « faible agitation », une agitation faite à une degré de cisaillement inférieur à 1000 $s^{-1}$ et de préférence inférieur à 100 $s^{-1}$.

En même temps que la phase F, on peut éventuellement ajouter le ou les parfums s'ils sont en quantité inférieure à 0,4 % du poids total de la composition. S'ils sont en quantités plus importantes, le surplus par rapport à 0,4 % est ajouté dans la phase huileuse. C'est ainsi que si la composition contient 1 % de parfum, 0,6 % sont dans la phase huileuse et 0,4 % sont dans la phase aqueuse (F).

Les compositions de l'invention peuvent être utilisées sur toutes matières kératiniques telles que la peau, le cuir chevelu, les cheveux, les cils, les sourcils, les ongles ou les muqueuses. Elles peuvent être utilisées comme produit de soin de la peau, par exemple comme crème de protection, de traitement ou de soin pour le visage, pour les mains ou pour le corps, comme lait corporel de protection ou de soin de la peau, du cuir chevelu ou des muqueuses, ou comme

produit d'hygiène, par exemple comme produit de nettoyage de la peau ou des muqueuses, ou encore comme produit capillaire ou comme produit solaire.

Les compositions peuvent constituer aussi des produits pour le maquillage de la peau et/ou des cheveux, par exemple en incorporant des pigments dans la composition pour constituer notamment des fonds de teint.

L'invention a encore pour objet l'utilisation cosmétique de la composition telle que définie ci-dessus comme produit de soin de la peau, comme produit d'hygiène, comme produit capillaire, comme produit solaire et comme produit de maquillage.

Un autre objet de l'invention est un procédé de traitement cosmétique d'une matière kératinique, telle que la peau, du cuir chevelu, des cheveux, des cils, des sourcils, des ongles ou des muqueuses, caractérisé par le fait qu'on applique sur la matière kératinique, une composition telle que définie ci-dessus.

Par ailleurs, quand les compositions selon l'invention ont la fluidité appropriée, elles peuvent servir aussi pour l'imprégnation de substrats insolubles dans l'eau pour constituer des articles (tels que lingettes) destinés au soin, au nettoyage et/ou au démaquillage de la peau, des cils et/ou des lèvres. Le substrat insoluble dans l'eau peut comprendre une ou plusieurs couches et il peut être choisi dans le groupe comprenant des matériaux tissés, des matériaux non-tissés, des mousses, des éponges, des ouates, en feuilles, boules ou films. Il peut s'agir notamment d'un substrat non tissé à base de fibres d'origine naturelle (lin, laine, coton, soie) ou d'origine synthétique (dérivés de cellulose, viscose, dérivés polyvinyliques, polyesters comme téréphtalate de polyéthylène, polyoléfines comme polyéthylène ou polypropylène, polyamides comme le Nylon, dérivés acryliques). Les non tissés sont décrits de façon générale dans RIEDEL « Nonwoven Bonding Methods & Materials », Nonwoven World (1987). Ces substrats sont obtenus selon les procédés usuels de la technique de préparation des non-tissés.

Quand le substrat est un non-tissé, on utilise de préférence un non-tissé épais, qui ne se met pas en boule et qui est assez solide pour ne pas se déliter et ne pas pelucher à l'application sur la peau. Il doit être absorbant, doux au moins sur une face pour le démaquillage des yeux en particulier. Comme non-tissés appropriés, on peut citer par exemple ceux commercialisés sous les dénominations Ultraloft 15285-01, Ultraloft 182-008, Ultraloft 182-010, Ultraloft 182-016 par la société BBA, Vilmed M1519 Blau, Vilmed M 1550 N et 112-132-3 par la société Freudenberg, celui commercialisé sous la dénomination Norafin 11601-010B par la société Jacob Holm Industries, les non tissés flockés commercialisés sous les dénominations Univel 109 et Univel 119 par la société Uni Flockage.

Par ailleurs, ce substrat peut comporter une ou plusieurs couches ayant des propriétés identiques ou différentes et avoir des propriétés d'élasticité, de douceur et autres appropriées à l'usage recherché. Les substrats peuvent comporter par exemple deux parties ayant des propriétés d'élasticité différentes comme décrit dans le document WO-A-99/13861 ou comporter une seule couche avec des densités différentes comme décrit dans le document WO-A-99/25318 ou comporter deux couches de textures différentes comme décrit dans le document WO-A-98/18441.

Les compositions imprégnées sur le substrat peuvent contenir tout composé approprié pour le but recherché, par exemple des tensioactifs moussants pour l'obtention de lingettes nettoyantes, ou des actifs de soin pour obtenir des lingettes de soin de la peau. On peut aussi les utiliser pour la maquillage de la peau, par exemple en imprégnant la lingette avec une composition contenant des pigments et pouvant constituer un fond de teint.

L'invention a donc aussi pour objet un article obtenu par imprégnation d'un substrat insoluble dans l'eau, avec une composition telle que définie ci-dessus.

L'invention a aussi pour objet l'utilisation de la composition telle que définie ci-dessus pour la préparation d'un article destiné au soin, au démaquillage, au nettoyage ou au maquillage de la peau, des lèvres et/ou des cils.

Les exemples suivants illustrent l'invention sans présenter un caractère limitatif. Les quantités y sont des pourcentages en poids. Les composés sont indiqués en nom INCI ou en noms chimiques selon les cas.

Exemples 1 à 3 selon l'invention

| | | Exemple 1 | Exemple 2 | Exemple 3 |
|---|---|---|---|---|
| PEG 8 isostearate | A | 5,80 | 5,80 | 5,80 |
| Polyisobutène hydrogéné | | 8,75 | 8,75 | 8,75 |
| Silicone cyclique | | 8,75 | 8,75 | 8,75 |
| Eau | B | 2,18 | 2,18 | 2,18 |
| Eau | D | 45,07 | 45,07 | 45,07 |
| Eau | F | Qsp 100 | Qsp 100 | Qsp 100 |

(suite)

| | | Exemple 1 | Exemple 2 | Exemple 3 |
|---|---|---|---|---|
| Conservateur | | 0,78 | 0,88 | 0,88 |
| Disodium stearoyl glutamate | | 0,16 | 0,16 | 0,16 |
| glycérine | | 3 | | |
| Steareth-100/PEG-136/HMDI Copolymer (SERAD FX1100) | | 0,4 | 0,2 | 0,4 |
| Acide phényl benzimidazole sulfonique | | 2 | | |
| Benzophénone 4 | | | 0,2 | |
| Méthylène bis-benzotriazolyl tetramethylbutylphénol | | | | 5 |
| Triéthanolamine | | 1,35 | 0,09 | 0,4 |
| Parfum | | 0,10 | 0,10 | 0,10 |
| | | | | |
| Diamètre des globules de la phase dispersée (mesurée par diffusion quasi élastique de la lumière)* | 24 h. | 87 nm | 97 nm | 165 nm |
| Viscosité à 25°C (Rhéomat 180) | | 12,8 Poises | 10,6 Poises | 12 poises |
| pH à 25°C | | 7,37 | 7,32 | 7,4 |
| Aspect visuel | | Opalescente | Opalescente | Blanche |
| Stabilité à différentes températures (4°, TA, 45°C) | | Stable | Stable | Stable |
| *La mesure est faite au BI-90 de Brookhaven. La taille des gouttelettes de phase huileuse est mesurée avant gélification par le polymère. | | | | |

Exemples comparatifs 4 et 5

| | | Exemple 4 | Exemple 5 |
|---|---|---|---|
| PEG 8 isostearate | A | 5,80 | 5,80 |
| Polyisobutène hydrogéné | | 8,75 | 8,75 |
| Silicone cyclique | | 8,75 | 8,75 |
| Ethyl hexyl méthoxycinnamate | | 7,5 | |
| Butyl methoxy dibenzoylmethane | | | 3 |
| Eau | B | 2,18 | 2,18 |
| Eau | D | 45,07 | 45,07 |
| Eau | F | Qsp 100 | Qsp 100 |
| Conservateur | | 0,78 | 0,78 |
| Disodium stearoyl glutamate | | 0,16 | 0,16 |
| Parfum | | 0,10 | 0,10 |
| | | | |
| Diamètre des globules de la phase dispersée (mesurée par diffusion quasi élastique de la lumière)* | 24 h | Non mesurée | Non mesurée |

(suite)

| | | Exemple 4 | Exemple 5 |
|---|---|---|---|
| Stabilité à différentes températures (4°, TA, 45°C) | | 2 phases après 4 heures à 25°C | 2 phases après 4 heures à 25°C |
| *La mesure est faite au BI-90 de Brookhaven. | | | |

Ces exemples se différencient des exemples selon l'invention du fait que les filtres utilisés sont des filtres lipophiles qui ne permettent pas de garder une bonne stabilité physicochimique des émulsions fines.

## Revendications

**1.** Composition pour application topique sous forme d'émulsion huile-dans-eau, comprenant une phase huileuse dispersée dans une phase aqueuse, dont les globules de phase huileuse ont un diamètre moyen allant de 50 à 500 nm, **caractérisée en ce que :**

- elle contient un système émulsionnant contenant (i) au moins un tensioactif non ionique, ayant un point de fusion inférieur à 45°C et un HLB allant de 10 à 15, ledit tensioactif comprenant une partie polaire comportant au moins 5 groupes oxyéthylénés et une partie apolaire comportant au moins une chaîne alkyle ramifiée ou insaturée, ayant de 14 à 22 atomes de carbone, et (ii) au moins un tensioactif anionique,
- elle contient au moins un filtre UV hydrophile,
- la phase huileuse contient des constituants huileux dont au moins une huile hydrocarbonée de poids moléculaire égal ou supérieur à 400, la quantité d'huiles hydrocarbonées de poids moléculaire égal ou supérieur à 400 représentant au moins 25 % en poids par rapport au poids total de la phase huileuse et la quantité d'huiles à base de triglycérides représentant moins de 15 % en poids par rapport au poids total de la phase huileuse,
- le rapport en poids de la quantité des constituants huileux de la phase huileuse sur la quantité du système émulsionnant va de 0,8 à 3,5.

**2.** Composition selon la revendication 1, **caractérisée en ce que** le tensioactif non ionique est choisi parmi les esters d'acide gras et de polyéthylène glycol, les esters de polyol et d'acide gras oxyéthylénés, les éthers d'alcool gras oxyéthylénés, et leurs mélanges.

**3.** Composition selon la revendication 1 ou 2, **caractérisée en ce que** le tensioactif non ionique est choisi parmi le PEG-8 isostearate, le PEG-10 isostearate, le PEG-12 isostearate, le PEG-15 isostearate, le PEG-15 glyceryl isostearate, le PEG-20 sorbitan triisostearate, l'isosteareth-10, et leurs mélanges.

**4.** Composition selon l'une quelconque des revendications précédentes, **caractérisée en ce que** la quantité de tensioactif(s) non ionique(s) va de 0,5 à 10 % en poids par rapport au poids total de la composition.

**5.** Composition selon l'une quelconque des revendications précédentes, **caractérisée en ce que** le tensioactif anionique est choisi dans le groupe formé par:

- les sels alcalins du dicetyl- et du dimyristylphosphate ;
- les sels alcalins du cholestérol sulfate ;
- les sels alcalins du cholestérol phosphate ;
- les sels d'acylaminoacides ;
- les sels de sodium de l'acide phosphatidique ;
- les alkyl sulfates et sulfonates ;
- et leurs mélanges.

**6.** Composition selon l'une quelconque des revendications précédentes, **caractérisée en ce que** la quantité de tensioactif(s) anionique(s) va de 0,05 à 2 % en poids par rapport au poids total de la composition.

**7.** Composition selon l'une quelconque des revendications précédentes, **caractérisée en ce que** la quantité de système émulsionnant va de 0,6 à 11 % en poids par rapport au poids total de la composition.

8. Composition selon l'une quelconque des revendications précédentes, **caractérisée en ce que** le filtre UV hydrophile est choisi parmi les filtres UV organiques, les agents filtrants, et leurs mélanges.

9. Composition selon l'une quelconque des revendications précédentes, **caractérisée en ce que** le filtre UV hydrophile est choisi parmi les dérivés du phényl benzimidazole, les dérivés de la benzophénone, les dérivés du phényl benzotriazole et leurs mélanges.

10. Composition selon l'une quelconque des revendications précédentes, **caractérisée en ce que** la quantité de filtre (s) UV hydrophile va de 0,1 à 20 % en poids par rapport au poids total de la composition.

11. Composition selon l'une quelconque des revendications précédentes, **caractérisée en ce qu**'au moins une huile hydrocarbonée de poids moléculaire égal ou supérieur à 400 est choisie parmi les alcanes ayant un point de fusion inférieur à 45°C.

12. Composition selon l'une quelconque des revendications précédentes, **caractérisée en ce que** la quantité de phase huileuse va de 0,5 à 55 % en poids par rapport au poids total de la composition.

13. Utilisation cosmétique de la composition selon l'une quelconque des revendications 1 à 12, comme produit de soin de la peau, comme produit d'hygiène, comme produit capillaire, comme produit solaire et comme produit de maquillage.

14. Procédé de traitement cosmétique d'une matière kératinique, **caractérisé en ce qu**'on applique sur la matière kératinique, une composition selon l'une quelconque des revendications 1 à 12.

15. Article obtenu par imprégnation d'un substrat insoluble dans l'eau, avec une composition selon l'une quelconque des revendications 1 à 12.

16. Utilisation de la composition selon l'une quelconque des revendications 1 à 12, pour la préparation d'un article destiné au soin, au démaquillage, au nettoyage ou au maquillage de la peau, des lèvres et/ou des cils.

17. Procédé de préparation de la composition selon l'une quelconque des revendications 1 à 12, consistant :

(1) à préparer la phase huileuse (A) contenant la ou les huiles et autres corps gras, et le système émulsionnant, sous agitation, à une température allant de 20°C à 45°C, jusqu'à obtention d'une phase homogène,
(2) à introduire dans la phase (A) 0.1 à 3 % en poids d'eau (phase B) par rapport au poids total de la composition, et à mélanger jusqu'à obtention d'une phase homogène (C),
(3) à ajouter dans la phase (C), 55 à 75% en poids d'eau (phase D) par rapport au poids total de la composition, pour obtenir après mélange une phase E homogène, et
(4) à ajouter le reste des constituants de la phase aqueuse (phase F).

18. Procédé selon la revendication précédente, **caractérisé en ce que** l'agitation est faite à un degré de cisaillement inférieur à 1000 $s^{-1}$.

## Claims

1. Composition for topical application in the form of an oil-in-water emulsion, comprising an oily phase dispersed in an aqueous phase, the oily phase globules of which have an average diameter ranging from 50 to 500 nm, **characterized in that**:

- it contains an emulsifying system containing (i) at least one nonionic surfactant having a melting point of less than 45°C and an HLB ranging from 10 to 15, said surfactant comprising a polar portion containing at least 5 oxyethylenated groups and an apolar portion containing at least one branched or unsaturated alkyl chain having from 14 to 22 carbon atoms, and (ii) at least one anionic surfactant,
- it contains at least one hydrophilic UV screen,
- the oily phase contains oily constituents including at least one hydrocarbon-based oil having a molecular weight of greater than or equal to 400, the amount of hydrocarbon-based oils having a molecular weight of greater than or equal to 400 representing at least 25% by weight relative to the total weight of the oily phase

and the amount of triglyceride-based oils representing less than 15% by weight relative to the total weight of the oily phase,
- the weight ratio of the amount of the oily constituents of the oily phase to the amount of the emulsifying system ranges from 0.8 to 3.5.

2. Composition according to Claim 1, **characterized in that** the nonionic surfactant is chosen from esters of fatty acid and of polyethylene glycol, oxyethylenated esters of a polyol and of a fatty acid, oxyethylenated fatty alcohol ethers, and mixtures thereof.

3. Composition according to Claim 1 or 2, **characterized in that** the nonionic surfactant is chosen from PEG-8 isostearate, PEG-10 isostearate, PEG-12 isostearate, PEG-15 isostearate, PEG-15 glyceryl isostearate, PEG-20 sorbitan triisostearate, isosteareth-10, and mixtures thereof.

4. Composition according to any one of the preceding claims, **characterized in that** the amount of nonionic surfactant (s) ranges from 0.5 to 10% by weight relative to the total weight of the composition.

5. Composition according to any one of the preceding claims, **characterized in that** the anionic surfactant is chosen from the group made up of:

   - alkali metal salts of dicetyl phosphate and of dimyristyl phosphate;
   - alkali metal salts of cholesterol sulphate;
   - alkali metal salts of cholesterol phosphate;
   - acylamino acid salts;
   - sodium salts of phosphatidic acid;
   - alkyl sulphates and alkyl sulphonates;
   - and mixtures thereof.

6. Composition according to any one of the preceding claims, **characterized in that** the amount of anionic surfactant (s) ranges from 0.05 to 2% by weight relative to the total weight of the composition.

7. Composition according to any one of the preceding claims, **characterized in that** the amount of emulsifying system ranges from 0.6 to 11% by weight relative to the total weight of the composition.

8. Composition according to any one of the preceding claims, **characterized in that** the hydrophilic UV screen is chosen from organic UV screens, screening agents, and mixtures thereof.

9. Composition according to any one of the preceding claims, **characterized in that** the hydrophilic UV screen is chosen from phenylbenzimidazole derivatives, benzophenone derivatives, phenylbenzotriazole derivatives, and mixtures thereof.

10. Composition according to any one of the preceding claims, **characterized in that** the amount of hydrophilic UV screen(s) ranges from 0.1 to 20% by weight relative to the total weight of the composition.

11. Composition according to any one of the preceding claims, **characterized in that** at least one hydrocarbon-based oil having a molecular weight of greater than or equal to 400 is chosen from alkanes having a melting point of less than 45°C.

12. Composition according to any one of the preceding claims, **characterized in that** the amount of oily phase ranges from 0.5 to 55% by weight relative to the total weight of the composition.

13. Cosmetic use of the composition according to any one of Claims 1 to 12, as a skincare product, as a hygiene product, as a hair product, as an antisun product and as a makeup product.

14. Process for cosmetic treatment of a keratin material, **characterized in that** a composition according to any one of Claims 1 to 12 is applied to the keratin material.

15. Item obtained by impregnation of a water-insoluble substrate with a composition according to any one of Claims 1 to 12.

**16.** Use of the composition according to any one of Claims 1 to 12, for the preparation of an item intended for caring for, removing makeup from, cleansing or making up the skin, the lips and/or the eyelashes.

**17.** Process for preparing the composition according to any one of Claims 1 to 12, consisting:

(1) in preparing the oily phase (A) containing the oil(s) and other fatty substances, and the emulsifying system, with stirring, at a temperature ranging from 20°C to 45°C, until a homogeneous phase is obtained,
(2) in introducing into the phase (A) 0.1 to 3% by weight of water (phase B) relative to the total weight of the composition, and in mixing until a homogeneous phase (C) is obtained,
(3) in adding, to the phase (C), 55 to 75% by weight of water (phase D) relative to the total weight of the composition, so as to obtain, after mixing, a homogeneous phase (E) and
(4) in adding the rest of the constituents of the aqueous phase (phase F).

**18.** Process according to the preceding claim, **characterized in that** the stirring is carried out with a degree of shear of less than 1000 $s^{-1}$.

**Patentansprüche**

**1.** Zusammensetzung für die topische Anwendung in Form einer Öl-in-Wasser-Emulsion, die eine in einer wässrigen Phase dispergierte Ölphase enthält, bei der die Kügelchen der Ölphase einen mittleren Durchmesser von 50 bis 500 nm aufweisen, **dadurch gekennzeichnet, dass**:

- sie ein Emulgatorsystem enthält, das (i) mindestens einen nichtionischen grenzflächenaktiven Stoff, der einen Schmelzpunkt unter 45 °C und einen HLB-Wert von 10 bis 15 aufweist, wobei der grenzflächenaktive Stoff einen polaren Bereich, der mindestens 5 Oxyethylengruppen enthält, und einen apolaren Bereich besitzt, der mindestens eine verzweigte oder ungesättigte Alkylkette mit 14 bis 22 Kohlenstoffatomen umfasst, und (ii) mindestens einen anionischen grenzflächenaktiven Stoff enthält,
- sie mindestens ein hydrophiles UV-Filter enthält,
- die Ölphase Ölbestandteile enthält, darunter mindestens ein Kohlenwasserstofföl mit einer Molmasse von 400 oder darüber, wobei der Mengenanteil der Kohlenwasserstofföle mit einer Molmasse von 400 oder darüber mindestens 25 Gew.-%, bezogen auf das Gesamtgewicht der Ölphase, ausmacht und der Mengenanteil der Öle auf der Basis von Triglyceriden weniger als 15 Gew.-%, bezogen auf das Gesamtgewicht der Ölphase, beträgt,
- das Gewichtsverhältnis der Menge der Ölbestandteile der Ölphase und der Menge des Emulgatorsystems im Bereich von 0,8 bis 3,5 liegt.

**2.** Zusammensetzung nach Anspruch 1, **dadurch gekennzeichnet, dass** der nichtionische grenzflächenaktive Stoff unter den Polyethylenglycolfettsäureestern, ethoxylierten Polyolfettsäureestern, ethoxylierten Fettalkoholethern und deren Gemischen ausgewählt ist.

**3.** Zusammensetzung nach Anspruch 1 oder 2, **dadurch gekennzeichnet, dass** der nichtionische grenzflächenaktive Stoff unter PEG 8-isostearat, PEG 10-isostearat, PEG 12-isostearat, PEG 15-isostearat, PEG 15-glycerylisostearat, PEG 20-sorbitantriisostearat, Isosteareth-10 und deren Gemischen ausgewählt ist.

**4.** Zusammensetzung nach einem der vorhergehenden Ansprüche, **dadurch gekennzeichnet, dass** der Mengenanteil des nichtionischen grenzflächenaktiven Stoffes oder der nichtionischen grenzflächenaktiven Stoffe im Bereich von 0,5 bis 10 Gew.-%, bezogen auf das Gesamtgewicht der Zusammensetzung, liegt.

**5.** Zusammensetzung nach einem der vorhergehenden Ansprüche, **dadurch gekennzeichnet, dass** der anionische grenzflächenaktive Stoff ausgewählt ist unter:

- Alkalisalzen von Dicetyl- und Dimyristylphosphat;
- Alkalisalzen von Cholesterinsulfat;
- Alkalisalzen von Cholesterinphosphat;
- Salzen von Acylaminosäuren;
- Natriumsalzen von Phosphatidsäure;
- Alkylsulfaten und -sulfonaten;

- und deren Gemischen.

6. Zusammensetzung nach einem der vorhergehenden Ansprüche, **dadurch gekennzeichnet, dass** der Mengenanteil des anionischen grenzflächenaktiven Stoffes oder der anionischen grenzflächenaktiven Stoffe im Bereich von 0,05 bis 2 Gew.-%, bezogen auf das Gesamtgewicht der Zusammensetzung, liegt.

7. Zusammensetzung nach einem der vorhergehenden Ansprüche, **dadurch gekennzeichnet, dass** der Mengenanteil des Emulgatorsystems im Bereich von 0,6 bis 11 Gew.-%, bezogen auf das Gesamtgewicht der Zusammensetzung, liegt.

8. Zusammensetzung nach einem der vorhergehenden Ansprüche, **dadurch gekennzeichnet, dass** das hydrophile UV-Filter unter den organischen UV-Filtern, filtrierenden Stoffen und deren Gemischen ausgewählt ist.

9. Zusammensetzung nach einem der vorhergehenden Ansprüche, **dadurch gekennzeichnet, dass** das hydrophile UV-Filter unter den Phenylbenzimidazolderivaten, Benzophenonderivaten, Phenylbenzotriazolderivaten und deren Gemischen ausgewählt ist.

10. Zusammensetzung nach einem der vorhergehenden Ansprüche, **dadurch gekennzeichnet, dass** der Mengenanteil des hydrophilen UV-Filters oder der hydrophilen UV-Filter im Bereich von 0,1 bis 20 Gew.-%, bezogen auf das Gesamtgewicht der Zusammensetzung, liegt.

11. Zusammensetzung nach einem der vorhergehenden Ansprüche, **dadurch gekennzeichnet, dass** mindestens ein Kohlenwasserstofföl mit einer Molmasse von 400 oder darüber unter den Alkanen mit einem Schmelzpunkt unter 45 °C ausgewählt ist.

12. Zusammensetzung nach einem der vorhergehenden Ansprüche, **dadurch gekennzeichnet, dass** der Mengenanteil der Ölphase im Bereich von 0,5 bis 55 Gew.-%, bezogen auf das Gesamtgewicht der Zusammensetzung, liegt.

13. Kosmetische Verwendung der Zusammensetzung nach einem der Ansprüche 1 bis 12 als Produkt für die Pflege der Haut, als Hygieneprodukt, als Produkt für die Haarbehandlung, als Sonnenschutzprodukt und als Produkt zum Schminken.

14. Verfahren zur kosmetischen Behandlung einer Keratinsubstanz, **dadurch gekennzeichnet, dass** auf die Keratinsubstanz eine Zusammensetzung nach einem der Ansprüche 1 bis 12 aufgetragen wird.

15. Gegenstand, der erhalten wird, indem ein in Wasser unlösliches Substrat mit einer Zusammensetzung nach einem der Ansprüche 1 bis 12 imprägniert wird.

16. Verwendung der Zusammensetzung nach einem der Ansprüche 1 bis 12 für die Herstellung eines Gegenstands, der für die Pflege, zum Abschminken, für die Reinigung oder zum Schminken der Haut, der Lippen und/ oder der Wimpern vorgesehen ist.

17. Verfahren zur Herstellung einer Zusammensetzung nach einem der Ansprüche von 1 bis 12, das darin besteht:

    (1) eine Ölphase (A), die das oder die Öle und weitere Fettsubstanzen und das Emulgatorsystem enthält, bei einer Temperatur von 20 bis 45 °C unter Rühren bis zur Bildung einer homogenen Phase herzustellen,
    (2) in die Phase (A) 0,1 bis 3 Gew.-% Wasser (Phase B), bezogen auf das Gesamtgewicht der Zusammensetzung, einzubringen und bis zur Bildung einer homogenen Phase (C) zu vermischen.
    (3) in die Phase (C) 55 bis 75 Gew.-% Wasser (Phase D), bezogen auf das Gesamtgewicht der Zusammensetzung, einzubringen, um nach dem Mischen eine homogene Phase (E) zu erhalten, und
    (4) die restlichen Bestandteile der wässrigen Phase (Phase F) zuzugeben.

18. Verfahren nach dem vorhergehenden Anspruch, **dadurch gekennzeichnet, dass** das Rühren bei einem Schergrad unter 1 000 $s^{-1}$ erfolgt.